# EUROPEAN PATENT APPLICATION

(11) **EP 4 803 604 A2**
(43) Date of publication of application: **09.09.2026**
(21) Application number: 26194063.9
(22) Date of filing: 12.02.2020
(51) Int. Cl.: C12M 1/00

(54) **ADJUSTABLE FERMENTATION AND CELL CULTURE FLASKS**

(30) Priority: 25.02.2019 US 201962809796 P
(62) Document number(s) of the earlier application(s) in accordance with Art. 76 EPC: 23186296.2 / 4 249 581; 20762774.6 / 3 931 298
(71) Applicant: University of Maryland, Baltimore County, Baltimore, MD 21250 (US)
(72) Inventor: RAO, Govind, Ellicott City, Maryland 21042 (US); KOSTOV, Yordan, Columbia, Maryland 21044 (US); TOLOSA, Michael, Columbia, Maryland 21046 (US); GE, Xudong, Woodstock, Maryland 21163 (US); KUMAR, Vikash, Catonsville, Maryland 21228 (US); RAHMATNEJAD, Vida, Baltimore, Maryland 21250 (US)
(74) Representative: ABG Intellectual Property Law, S.L.

(57) **Abstract**

The present invention relates to collapsible and/or foldable fermentation and/or culture vessels fabricated of an adjustable frame further including sections of gas permeable material or in the alternative a non-rigid gas permeable polymeric container or bag fabricated of either a gas permeable or non- gas permeable material with the further benefit of more efficient inventory space and disposal space.

## Description

### CROSS REFERENCE TO RELATED APPLICATION

This application claims priority to U.S. Provisional Patent Application No. 62/809,796 filed on February 25, 2019, the contents of which are hereby incorporated by reference herein for all purposes.

### Technical Field

The present invention relates to collapsible and/or foldable fermentation and/or culture vessels fabricated of gas permeable or non-gas permeable material and may include an adjustable frame further including sections of gas permeable material or in the alternative a non-rigid gas permeable polymeric container or bag fabricated of a gas permeable material for gas exchange with the further benefit of more efficient inventory space, disposal space and can be used as alternatives or replacement of rigid-wall containers, such as those made of glass, metal or a rigid polymer.

### BACKGROUND OF THE INVENTION

### Related Art

The culture of cells is a critical element of biotechnology. Cells are cultured in small quantities during the research stage, and typically the magnitude of the culture increases as the research moves towards its objective of benefiting human and animal health care. The culture or processing of cells typically requires the use of a device to hold the cells, for example in an appropriate culture medium when culturing the cells. Certain devices and methods have become well established for research stage cell culture because they allow a wide variety of cell types to be cultured and are therefore useful to the widest audience. The known devices include shaker flasks, roller bottles, T-flasks and bags. Such devices are widely used but suffer from several drawbacks, including the material they are made of, such as glass, metal, or hard plastic vessels. Such vessels are expensive and require maintenance, as they are not disposable or sterile. In order to maintain a sterile or aseptic environment for cell culture, the vessels require sterilization, usually by autoclave. Therefore, the cell culture vessels must be washed and sterilized prior to and/or subsequent to their use. In addition, because glass and hard plastic cell culture vessels are not usually disposable, it is necessary to have adequate space for storage of such vessels. Thus, as glass, metal, and hard plastic cell culture vessels are expensive, not disposable, and require extensive maintenance, there is a need for cell culture vessels that are inexpensive, disposable, collapsible, and pre-sterilized.

### SUMMARY OF THE INVENTION

The present invention provides for different embodiments of collapsible or foldable vessels fabricated of a semi-rigid material and providing a frame for a fluid reservoir.

In one aspect, the present invention provides for a conical shaped flask comprising:
a conical shaped frame comprised of two rigid rod-type supports each having proximal end forming a flat base and two opposing upwardly extending distal ends to form a generally conical body, wherein the flat base of the two rigid supports are connected at a central pivot point thereby allowing the two rigid rod-type supports to form a conical body portion when separated furthest from each other and foldable into an essentially flat frame when adjacent;
a rigid circular ring cap forming a tubular neck for placement and connecting thereto the upwardly extending distal ends to stabilize the conical shaped frame, wherein the rigid circular ring cap has an exterior and interior edge; and
a non-rigid gas permeable polymeric container or bag fabricated of a gas permeable polymeric material and sized to fit within the conical body portion and attached to the interior edge of the circular ring cap for positioning therein.

Notably, the rigid circular ring cap preferably comprises four recesses/slots equally distributed within the bottom of the circular ring cap for insertion and stabilization of the upwardly extending distal ends. Further, the two rigid rod-type supports preferably have hook type attachments for securing the non-rigid gas permeable polymeric container or bag to the conical body portion, thereby providing the full extent of the fluid capacity of the polymeric container or bag. The rigid circular ring cap may further comprise a top stopper or a rigid screw type cap for closure of the non-rigid gas permeable polymeric container or bag. Further, the circular ring cap can be fabricated as a cylindrical ring to form a neck wherein the neck has a uniform internal diameter for its entire height. The height of the neck can be from about ½" to about 2". The interior diameter of the rigid circular ring cap is of a sufficient size for ease of transfer of fluids into and out of the polymeric bag. Additionally, this rigid ring can be threaded to provide for a screw on top to ensure closure of the flask.

In yet another aspect, the present invention relates to a T-flask that provides the advantages of being disposable, collapsible, and pre-sterilized.

A T-flask of the present invention comprises:
four rectangular frames each comprising four rigid support members pivotably attached to each other by corner hinges to form a two dimensional rectangular shape, wherein the four rigid support members are fabricated of a rigid material or substantially rigid material such as a polymer, metal or the like, wherein the four rectangular frames are connected through the corner hinges and pivot with respect to each other to an expanded configuration to form a three dimensional rectangular structure and a collapsed configuration wherein the rectangular frames lie approximately parallel to each other, wherein the hinges include locking mechanism to support the frame in expanded configuration; and
a non-rigid gas permeable polymeric container or bag comprising a chamber, a neck connected to the chamber for introducing fluids into the chamber, a closure cap attachable to the neck, wherein the chamber is sized to fit within the three dimensional rectangular structure, wherein the chamber of the non-rigid gas permeable polymeric container or bag is communicatively connected to the four rectangular frames, and wherein the neck and closure cap are not positioned within the rectangular frame.

In a further aspect, the present invention a collapsible flask comprising:
an Erlenmeyer flask shaped vessel fabricated of a gas permeable polymeric film comprising: a flat base, a generally conical body portion, a generally tubular neck having a diameter less than the body portion, wherein the tubular neck comprises an opening for moving fluid into and out of the vessel, wherein gas permeable polymeric film is sufficiently strong to maintain shape of the vessel and sufficiently flexible to allow collapsibility of the vessel. Optionally the tubular neck of the vessel may be provided with a rigid sealable cap and the tubular neck can include a threaded area for a screw on cap.

The collapsibility of the vessel is easy effected by collapsing the top section into the middle section and both of same into the bottom section of the vessel when the vessel is in an upright position. Thus, the sections of the vessel are collapsed along a vertical plane to form a lateral configuration. In the compressed or collapsed position, the vessel is in a better arrangement for storage or transport. The vessel can easily be opened into an expanded vessel by simply moving the top and middle section away from the bottom section.

The gas permeable polymeric film provides adequate rates of carbon dioxide and oxygen permeability while preventing passage of liquid. Several gas-permeable materials have gas permeability sufficient to permit free passage of oxygen and carbon dioxide and can be selected from a group including, but not limited to, silicone, fluoroethylenepolypropylene, polyolefin, polyethylene, ethylene vinyl acetate copolymer, a cellulose acetate, a methacrylate, a phthalate or a hybrid material such as the combination of nylon and silicone. Hybrid materials are formed by two or more components and combine the intrinsic characteristics of its individual constituents to additional properties due to synergistic effects between the components. Thus, the properties of hybrid nanomaterials can be tuned by changing their composition and morphology, leading to materials with enhanced performance characteristics, such as high thermal stability, mechanical strength, light emission, gas permeability, electron conductivity, and controlled wetting features.

In another aspect, the present invention provides a collapsible flask comprising:
an Erlenmeyer flask shaped vessel fabricated of both a gas permeable and a non-gas permeable polymeric film comprising: a flat base, a generally conical body portion, a generally tubular neck having a diameter less than the body portion and a mouth of the tubular neck for moving fluid into and out of the vessel, wherein the generally conical body portion comprises strips of connecting gas permeable and non-gas permeable polymeric sections, wherein the non-gas permeable sections provide sufficient support for maintaining the conical shape and the gas permeable polymeric sections cover a greater area from that of the non-gas permeable polymeric sections. Both the gas permeable and non-gas permeable polymeric films are sufficiently strong to maintain shape of the vessel and sufficiently flexible to allow collapsibility of the vessel. Optionally the tubular neck and mouth of the vessel may be provided with a rigid sealable cap, wherein the tubular neck can include a threaded section to provide closure with a screw on cap.

The collapsibility of the vessel is easy effected by collapsing the top section into the middle section and both in the bottom section of the vessel when the vessel is in an upright position. Thus, the sections of the vessel are collapsed vertically to form a compressed configuration. In the compressed or collapsed position, the vessel is in a better arrangement for storage or transport. The vessel can easily be compressed and opened into an expanded vessel by simply moving the top, and middle section away from the bottom section.

The gas permeable polymeric film provides adequate rates of carbon dioxide and oxygen permeability while preventing passage of liquid. Several gas-permeable materials have gas permeability sufficient to permit free passage of oxygen and carbon dioxide and can be selected from a group including, but not limited to, silicone, fluoroethylenepolypropylene, polyolefin, ethylene vinyl acetate copolymer, a cellulose acetate, a methacrylate, a hybrid material or a phthalate. The non-gas permeable sections can be any plastic commonly used in traditional culture vessels, or any other cell attachment material known to those skilled in the art.

In a still further aspect, the present invention provides for an inflatable flask structure comprising:
a plurality of pneumatically interconnected, elongate inflatable tubes positioned in spaced-apart relation to provide a conical shaped structure for being inflated in unison, said tubes defining a flask type structure have an opening at the proximal end and flat bottom structure at the distal end;
valve means for inflating the tubes; and
optionally a plurality of wall panels attached from and between adjacent tubes to define an enclosure of the flask, wherein the plurality of wall panels is fabricated from a gas permeable polymeric material and wherein the inflatable tubes are fabricated from an impermeable and flexible polymeric material.

The inflatable tubes are preferably fabricated of a flexible gas impermeable material such as a rubberized material, polymeric material or a thermoplastic sheet material, wherein the material has sufficient density to resist passage of air under pressure. When inflated the flask sharped structure is formed. Valve means are provided for inflating the tubes, wherein the valve means includes a manifold into which all of the tubes interconnect and the tubes are connected with an air pump and the tubes.

To provide a supporting system for the above inventive structures, the present invention further provides for an inflatable holder for supporting a flask comprising:
a flexible bag body having an open end wherein the open end comprises an adjustable valve for introducing compressed air into the flexible bag, wherein the adjustable valve is sealable to retain the compressed air after inflating, wherein the bag is sized sufficiently for providing an inflatable extendible rim to encompass and provide support for an inserted flask.

In another aspect, the present invention provides for a collapsible conical shaped Erlenmeyer flask comprising three sections, wherein the first section is a non-flexible neck section, the second section comprises a non-flexible flat bottom bowl section and a third section comprising a flexible sleeve that is positioned between the first and second section and connected to each to form a sealed collapsible Erlenmeyer flask, wherein the flexible sleeve collapsably folds upon itself to position the non-flexible neck section closer to the non-flexible flat bottom bowl section. Importantly, the flexible sleeve is fabricated of a gas permeable membrane, as described herein below and preferably a silicone material. In the folding process, the neck section and substantially all of the flexible sleeve are contained with the non-flexible flat bottom bowl section, thereby reducing the size for easy storage.

The flexible sleeve comprises a first opening having smaller cross-section diameter than a second opening, wherein the first and second opening are positioned at opposite ends of the flexible sleeve and where in the first opening is connect to the non-flexible neck section and the second opening is connect to the non-flexible flat bottom bowl section. The flexible sleeve is connected to both the non-flexible neck section and the non-flexible flat bottom bowl section by meeting the edges of each and overlapping a bottom section of the non-flexible neck section and the top section of the non-flexible flat bottom bowl section. The flexible sleeve has the ability and elasticity to stretch over the bottom edge of the neck section and the top edge of the non-flexible flat bottom bowl section. Additionally, a sealing band can be connected to flexible sleeve at the junction point of the overlapping sleeve section to the neck section and flat bottom bowl section. The sealing bands may be a narrow metal strip, wherein the bands can be slipped over the neck section for positioning at the appropriate junction points. The non-flexible neck section can include a threaded section to provide the option of a screwable cap for closure of the flask.

The flexible sleeve is fabricated from a gas permeable silicone type material. Silicone films may be less than about 3 mm, about 2 mm, about 1 mm, or about 0.8 mm in the surface areas where gas transfer is desired. The best selection of material and thickness depends on the application, however, preferably silicone rubber has been found to be most effective for bacteria growth because bacteria grown in the silicone rubber pouches or bags can readily take up oxygen and release waste gases, such as carbon dioxide. Notably, silicone rubbers are elastomers based on high molecular weight linear polymers, generally polydimethysiloxanes, which also may be modified with functional groups.

Additional features and advantages of the subject matter of the present disclosure will be set forth in the detailed description which follows, and in part will be readily apparent to those skilled in the art from that description or recognized by practicing the subject matter of the present disclosure as described herein, including the detailed description which follows, the claims, as well as the appended drawings

### BRIEF DESCRIPTION OF THE FIGURES

Figure 1 A illustrates a foldable support frame in an expanded configuration according to one embodiment of the invention.
Figure 1 B illustrates an example of a collapsible bag in expanded configuration, according to one embodiment of the invention.
Figure 2 A illustrates an example of a rigid cap, according to one embodiment of the invention.
Figure 2 B illustrates rigid cap of Figure 2 A connected to the support frame of Figure 1 A.
Figure 3 A illustrates the frame support of Figure 1 A and the collapsible bag of Figure 1B.
Figure 3 B illustrates the frame support of Figure 1 A in a foldable position.
Figure 4 illustrates a foldable support frame in an expanded configuration according to one embodiment of the invention.
Figure 5 illustrates a foldable support frame in an expanded configuration according to one embodiment of the invention and a rigid cap for connected to a neck section.
Figure 6 A illustrates a three-dimensional support frame according to one embodiment of the invention.
Figure 6 B illustrates the support frame of Figure 6 a collapsed into a folded frame.
Figure 7 illustrates an example of a collapsible bag in expanded configuration for inclusion in the support frame of Figure 6 A.
Figure 8 A illustrates an example of a collapsible flask in an expanded configuration according to one embodiment of the invention.
Figure 8 B illustrates the flask of Figure 8 A in a collapsed configuration.
Figure 9 A illustrates an example of a collapsible flask in an expanded configuration according to one embodiment of the invention.
Figure 9 B illustrates the flask of Figure 9 A in a collapsed configuration.
Figure 10 illustrates an inflatable support holder for stabilizing a flask of the present invention.
Figure 11 A illustrates an inflatable flask with gas permeable inserts to hold a liquid whererin gases escape from gas permeable inserts.
Figure 11 B illustrates an inflatable flask with inflatable tubes forming a conical structure.
Figure 11 C illustrates the inflatable flask of Figure 11A in a collapsed form.
Figure 12 illustrates a collapsible flask comprising three sections.
Figure 13 illustrates the collapsible flask of Figure 12 in a folded and collapsed position.
Figure 14 shows the calibration curve for DCO₂ probes.
Figure 15 DO curve for 0.3mm silicone thickness (blue) and solid walled (orange) shake flask variants for *E.Coli* growth in LB broth.
Figure 16 DCO₂ curve for 0.3mm silicone thickness (blue) and solid walled (orange) shake flask variants for *E.Coli* growth in LB broth.

### DETAILED DISCLOSURE OF THE INVENTION

Various embodiments of the disclosure will be described in detail with reference to drawings. Reference to various embodiments does not limit the scope of the invention. Additionally, any examples set forth in this specification are not limiting and merely set forth some of the many possible embodiments of the claimed invention. Like numbers used in the figures refer to like components, steps and the like.

All scientific and technical terms used herein have meanings commonly used in the art unless otherwise specified. The definitions provided herein are to facilitate understanding of certain terms used frequently herein and are not meant to limit the scope of the present disclosure.

"Include," "includes," or like terms means encompassing but not limited to, that is, inclusive and not exclusive.

"Optional" or "optionally" means that the subsequently described step, feature, condition, characteristic, or structure, occurs/is present or does not occur/is not present, while still being within the scope described.

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful and is not intended to exclude other embodiments from the scope of the inventive technology.

The vessels or flasks, the methods of making the vessels or flasks, and the method of using the vessels or flasks, described herein may include components or steps described herein, plus other components or steps not described herein.

As used in this specification and the appended claims, the term "or" is generally employed in its sense including "and/or" unless the content clearly dictates otherwise. The term "and/or" means one or all of the listed elements or a combination of any two or more of the listed elements.

As used herein, "have", "has", "having", "include", "includes", "including", "comprise", "comprises", "comprising" or the like are used in their open-ended inclusive sense, and generally mean "include, but not limited to", "includes, but not limited to", or "including, but not limited to".

Any direction referred to herein, such as "top," "bottom," "left," "right," "upper," "lower," "above," below," and other directions and orientations are described herein for clarity in reference to the figures and are not to be limiting of an actual device or system or use of the device or system. Many of the devices, articles or systems described herein may be used in a number of directions and orientations. Directional descriptors used herein with regard to cell culture vessels often refer to directions when the vessel is oriented for purposes of culturing cells in the apparatus.

While various features, elements or steps of particular embodiments may be disclosed using the transitional phrase "comprising," it is to be understood that alternative embodiments, including those that may be described using the transitional phrases "consisting" or "consisting essentially of" are implied.

The present invention provides for different embodiments of culture vessels or flasks that provide for gas permeability and structural integrity while also providing for vessels or flasks that are foldable and collapsible to provide for space-conscious vessels that are when stored occupy considerably less space than traditional rigid vessels or flasks. Some embodiments include at least a collapsible polymeric gas permeable container or bag and may differ depending upon the intended use but may have up to or greater than a 5 Liter capacity in some embodiments, or as small as 25 mL capacity or even smaller in other embodiments. Various example vessels or flasks may further include additional support members to contact and support the non-rigid gas permeable polymeric container or bag when in expanded configuration.

According to one embodiment, the non-rigid polymeric containers or bags are collapsible to lie substantially flat, or at least to a reduced profile, and expandable to define a volume when in use. The non-rigid gas permeable polymeric containers or bags may be constructed from flexible, non-rigid materials, having an open end and a closed end. The non-rigid gas permeable polymeric containers or bags may be foldable or otherwise collapsible for storage. The non-rigid gas permeable polymeric containers or bags may be constructed in any suitable configuration that would define the desired shape and volume when expanded for use.

For example, according to one embodiment, a non-rigid gas permeable polymeric container or bag may be formed as a single sheet using thermal forming techniques or blow molding techniques. According to another embodiment, however, the non-rigid gas permeable polymeric container or bag may be formed from two sheets (having any preformed shape) mated together at or near the edges, using any suitable mating technique, such as, but not limited to, solvent welding, radio frequency ("RF") welding, sonic welding, heat sealing, adhesives, and the like. The open end of the non-rigid gas permeable polymeric container or bag may be permanently or removably sealed to the underneath side of the rigid cap, or may be sealed to the outer edge of the rigid cap. According to one embodiment, the closed end of the container or bag can be formed in several different shapes to ensure that the fluid contained within the container or bag does not pool or gather along the bottom.

Examples of collapsible containers or bags may include a rigid cap to which the non-rigid gas permeable polymeric container or bag is attached. The rigid cap assists in defining the open space and cross-sectional geometry of the container by defining the shape of the open end. The geometry of the rigid cap may vary, according to various embodiments described herein. For example, in one embodiment the rigid cap may have a substantially circular geometry, whereas in other embodiments the rigid cap may be more elongated, ovular, or elliptical in shape. In yet another embodiment, the rigid cap may be formed as a long, narrow cap, having a reduced width that is just wide enough to house one or more ports.

Any number of fittings may be used to provide a fluid inlet, vent, or vacuum port, such as, but not limited to, barbed fitting female/male luer loc fitting, straight fitting, relief valve, one-way valve, and the like. The selection of the fitting will depend upon the intended use of the port. For example, a relief valve or one-way positive check valve may be used as a venting port, whereas a barbed fitting or female luer loc fitting may be used as an inlet port. According to one embodiment, the rigid cap may also have one or more fluid filters for filtering debris and other materials from the fluid flowing therethrough.

In some example embodiments, additional support members may also be included to support the non-rigid gas permeable polymeric container or bag when in expanded configuration. For example, the support members may be affixed to multiple points on the exterior surface of the non-rigid gas permeable polymeric container or bag, such that when the support members are expanded, they positively expand the non-rigid gas permeable polymeric container or bag to assist in defining the form, shape, and rigidity of the container or bag during use. These support members may be collapsible or otherwise deconstructed to also lie in a substantially flat configuration, or have a reduced profile, when not in use. In another example, the support members may be one or more adjustable support arms that are attachable to the rigid cap providing a tension to expand the non-rigid gas permeable polymeric container or bag in a direction away from the rigid cap. Any other suitable configuration of rigid support members that are collapsible and expandable to support the non-rigid gas permeable polymeric container or bag may be used with any of the example embodiments of collapsible fluid vessels or flasks described herein.

In some versions, the non-rigid gas permeable polymeric container or bag can be affixed to the support frame in a manner, such as by loops, clips, etc., as described more fully below, to resist the forces created by negative pressure which may otherwise cause the container to separate from the support frame and collapse inward.

The non-gas permeable polymeric material for supporting the described vessel or flasks are formed of any suitable material. Preferably, materials intended to contact cells or culture media are compatible with the cells and the media. Typically, cell culture components are formed from polymeric material. Examples of suitable polymeric materials include polystyrene, polymethylmethacrylate, polyvinyl chloride, polycarbonate, polysulfone, polystyrene copolymers, fluoropolymers, polyesters, polyamides, polystyrene butadiene copolymers, fully hydrogenated styrenic polymers, polycarbonate PDMS copolymers, and polyolefins such as polyethylene, polypropylene, polymethyl pentene, polypropylene copolymers and cyclic olefin copolymers, and the like.

Cell culture containers or bags that are constructed with gas permeable films advantageously provide a large surface area for gas exchange while maintaining a closed system. Disposables bags also helps reduce the risk of contamination for the cell culture and for the environment. Gas permeable films should be selected based on a variety of characteristics including gas permeability, moisture vapor transmission, capacity to be altered for desired cell interaction with cells, optical clarity, physical strength, and the like. A wide variety of information exists that describe the types of gas permeable materials that have been successfully used for cell culture.

Silicone films, made of silicone rubber, and used for cell culture bags have high oxygen and carbon dioxide permeability, good optical clarity, good resistance to puncture, typically do not bind cells, and can be easily fabricated into a wide variety of shapes. Silicone films may be less than about 3 mm, about 2 mm, about 1 mm, or about 0.8 mm in the surface areas where gas transfer is desired. The best selection of material and thickness depends on the application, however, preferably silicone rubber has been found to be most effective for bacteria growth because bacteria grown in the silicone rubber pouches or bags can readily take up oxygen and release waste gases, such as carbon dioxide. Notably, silicone rubbers are elastomers based on high molecular weight linear polymers, generally polydimethysiloxanes, which also may be modified with functional groups.

Fluoropolymer films have desirable characteristics that make them a popular choice for culture bags. Fluoropolymer films are considered biologically, chemically and immunologically inert, as well as being hydrophobic. Further, fluoropolymer films like FEP (fluorinated ethylene-propylene) do not trigger immune responses in immune cells and progenitor immune cells. Preferred fluoropolymer films include fluorinated ethylene-propylene (FEP), polytetrafluoroethylene (PTFE), 3M^{™} Dyneon^{™} TFM^{™} modified PTFE, polyvinylidenefluoride (PVDF), tetrafluoroethylene-perfluoro(propyl vinyl ether) (PFA), polyvinylidene difluoride (PVF), polychlorotrifluoroethlylene (PCTFE), tetrafluoroethylene/hexafluoropropylene/ethylene copolymer (HTE), chlorotrifluoroethylene/vinylidenefluoride copolymer, chlorotrifluoroethylene/hexafluoropropylene, ethylene/chlorotrifluoroethylene copolymers (ECTFE), ethylene/trifluoroethylene copolymers, ethylene/tetrafluoroethylene copolymers (ETFE), tetrafluoroethylene/propylene copolymers (TFE/P), tetrafluoroethylene/hexafluoropropylene copolymers (FEP/HFP), hexafluoropropylene/tetrafluoroethylene/vinylidene copolymer (THV), or perfluoro(1-butenyl vinyl ether) homocyclopolymer having functionalized polymer-end groups.

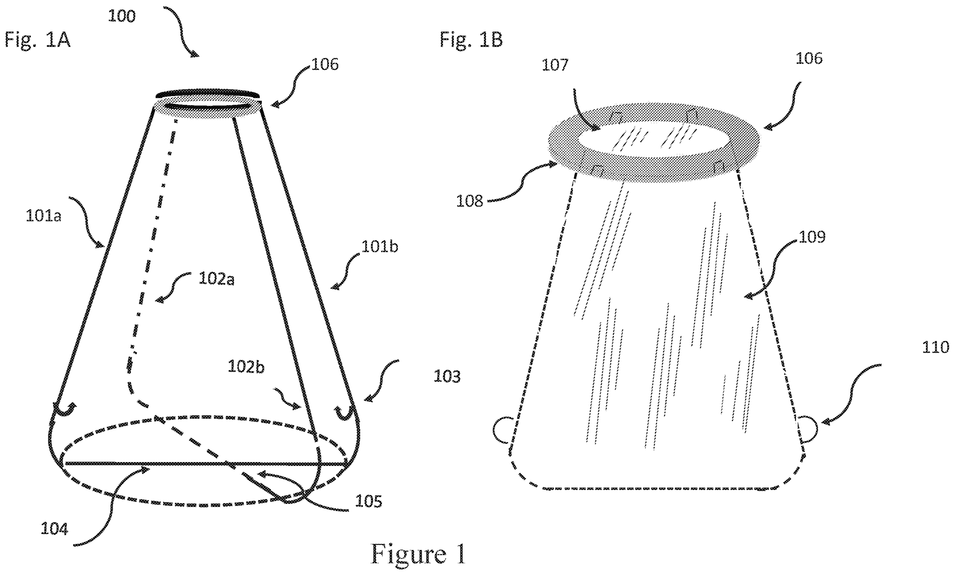

Turning now to a discussion of the drawings, the collapsible reservoir may be further understood with reference to Figures 1-16, providing examples of non-limiting embodiments.

**Figure 1A** illustrates a conical support frame **100** in an expanded configuration which includes two rigid rod-type supports **101** and **102** each having proximal end forming a flat base and two opposing upwardly extending distal ends **101a** and **101b** and **102a** and **102b** to form a generally conical body, wherein the flat base **104** of the two rigid supports are connected at a central pivot point **105** thereby allowing the two rigid rod-type supports to form a conical body portion when separated furthest from each other. The conical support frame **100** further comprises a rigid circular ring cap **106** forming a tubular neck for placement and connecting thereto the upwardly extending distal ends **101a** and **101b** and **102a** and **102b** to stabilize the conical shaped frame. The conical support frame **100** and circular cap **106** may be made from any rigid or substantially rigid polymeric materials described herein or otherwise suitable for such purposes, such as polyethylene (high-density or low-density polyethylene HDPE or LDPE), polyvinylchloride (PVC), polypropylene (PP), polystyrene (PS) including high impact polystyrene, polyamides (PA), acrylonitrile butadiene styrene (ABS), polycarbonate (PC), polycarbonate/acrylonitrile butadiene styrene (PC/ABS) etc. The upwardly extending distal ends **101a** and **101b** and **102a** and **102b** of the rigid rod can be configured in shapes including circular, triangular, rectangular and cubic. Notably, the outside corners of the frame are curved. Such curved corners provide for easy fitting into flask clamp system and platform that are commercially available to hold Erlenmeyer flask and provide stabilization during any shaking of the flask.

**Figure 1B** illustrates the rigid circular ring cap **106** having an exterior edge **108** and interior **107** edge; and a non-rigid gas permeable polymeric bag **109** fabricated of a gas permeable polymeric material and sized to fit within the conical body portion and attached to the interior edge **107** of the circular ring cap for positioning therein. The gas permeable polymeric bag is shown extended but is collapsible when inserting into the conical support frame **100**. Optionally the gas permeable polymeric bag can include ring type attachments **110** that can be connect to hooks **103** on the support frame **100** to extend the gas permeable bag when in the conical support frame **100**. Preferably the gas permeable polymeric bag is fabricated from a material that permits free passage of oxygen and carbon dioxide and can be selected from a group including, but not limited to, a silicone compounds such as polydimethylsiloxane (PDMS), poly1-trimethylsilyl-1-propyne (PMSP), polypropylmethylsiloxane, polytrifluoropropylmethylsiloxane, and polyphenylmethylsiloxane; fluoroethylenepolypropylene, polyolefin, ethylene vinyl acetate copolymer, polyethylene, a cellulose acetate, a methacrylate, a hybrid material or a phthalate. Preferably, a silicone compound is used to fabricate the pouch.

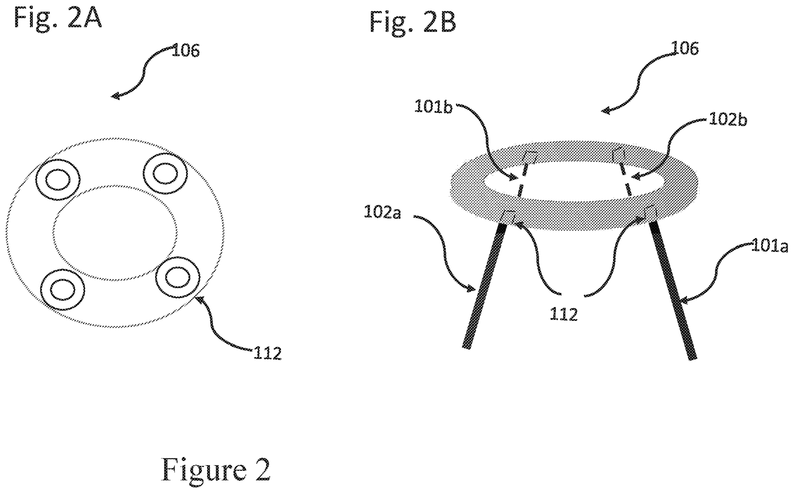

**Figure 2** **A** illustrates the rigid circular ring cap **106** with four equally separated recesses **112** that hold and provide support for the upwardly extending distal ends **101a** and **101b** and **102a** and **102b** to stabilize the conical shaped frame. The rigid circular ring cap **106** has sufficient size such as depth of material to include the recesses so the recesses are deep enough to hold the distal ends **101a** and **101b** and **102a** which can be constructed with a click in or locking connection. **Figure 2** **B** shows the upwardly extending distal ends **101a** and **101b** and **102a** locked in the recesses **112** of the rigid circular ring cap.

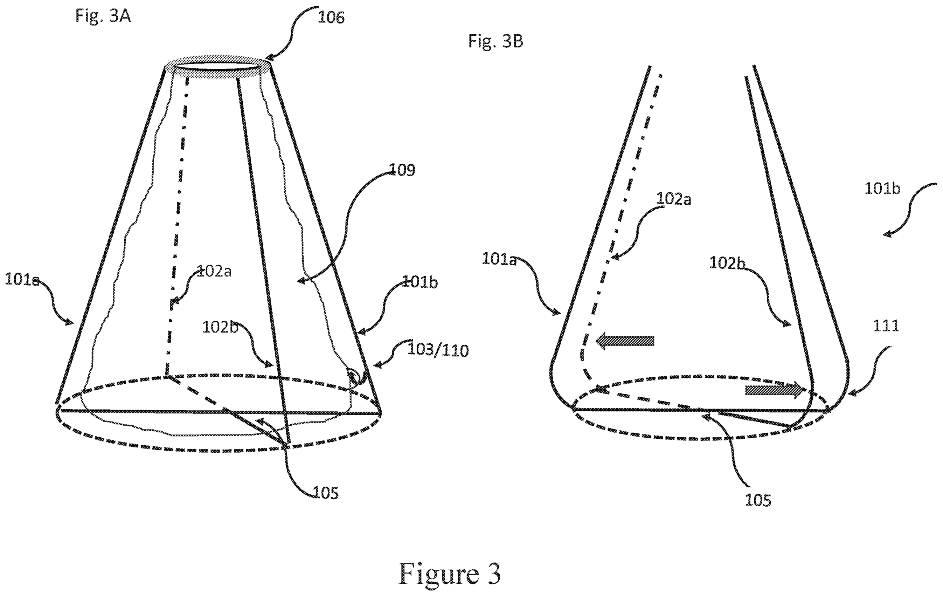

**Figure 3** **A** illustrates the conical support frame **100** including the rigid circular ring cap **106** attached to the gas permeable polymeric bag **109**. The gas permeable polymeric bag **109** is attached to the upwardly extending distal ends **101a** and **101b** and **102a** and **102b** by the interconnection between hook **103** and rings **110** thereby extending and stabilizing the gas permeable polymeric bag **109** when being used, such as filling or during growth of any cell cultures.

**Figure 3** **B** show one of the most important aspect of the present invention, that being, the ability to remove the gas permeable polymeric bag **109** and the rigid circular ring cap **106** for folding of the conical support frame **100**, wherein the upwardly extending distal ends **102a** and **102b** are moved on pivoting point **105** towards **101b** and **101a** to form a significantly flat configuration. Such foldability provides for easy storage. Notably, this **Figure 3B** also shows another form of the conical support frame wherein the outside corners of the frame are curved. Such a curved corner **111** provides for easy fitting into flask clamp system commercially available to hold Erlenmeyer flask and provide stabilization during any shaking of the flask.

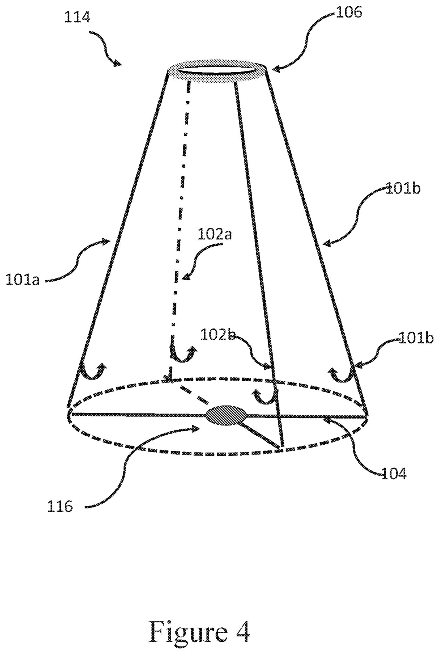

**Figure 4** illustrates another embodiment showing conical support frame **114** including rod-type supports **101** and **102** each having proximal end forming a flat base and two opposing upwardly extending distal ends **101a** and **101b** and **102a** and **102b** to form a generally conical body, wherein the flat base **104** of the two rigid supports is split and connected at a central pivot point **116** thereby allowing the two rigid rod-type supports to form a conical body portion when separated furthest from each other. In this embodiment the flat sections of each rod-type support are split in the middle of the flat base section and individually inserted into a central pivoting central unit **116**. This is different from the configuration of **Figure 1A** where in rod **101** is position above rod **102** on a different horizontal plane. In contrast in the **Figure 4**, the flat base of both rods **101** and **102** are positioned on the same horizontal plane. Again, this conical support frame **114** comprises a rigid circular ring cap **106** forming a tubular neck for placement and connecting thereto the upwardly extending distal ends **101a** and **101b** and **102a** and **102b** to stabilize the conical shaped frame.

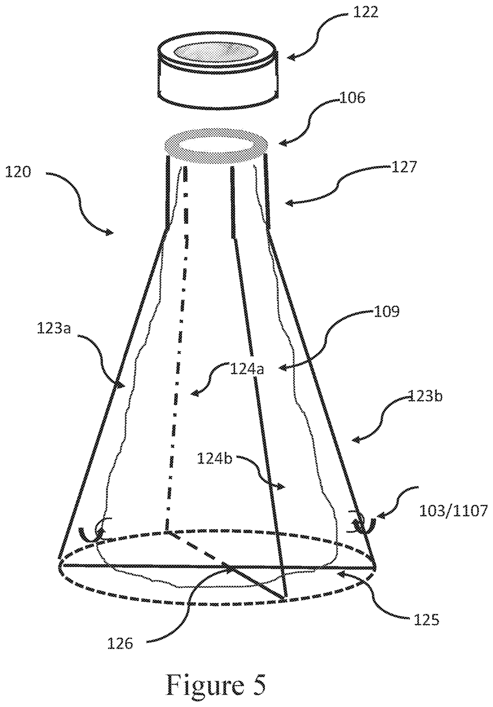

**Figure 5** illustrates another embodiment of a foldable Erlenmeyer shaped flask frame **120**, in an expanded configuration which includes two rigid rod-type supports **123** and **124** each having proximal end forming a flat base and two opposing upwardly extending distal ends **123a** and **123b** and **124a** and **124b** to form a generally conical body, wherein the two rigid rod-type supports **123** and **124** extend vertically at an angle of about **150** to **170** degrees relative to the position of the **123** and **124** rods to form a tubular neck having a diameter less than the body portion, wherein the flat base **125** of the two rigid supports are connected at a central pivot point **125** thereby allowing the two rigid rod-type supports to form a conical body portion when separated furthest from each other. The Erlenmeyer shaped flask frame **120** further comprises a rigid circular ring cap **106** forming a tubular neck for placement and connecting thereto the upwardly extending distal ends **123a** and **123b** and **124a** and **124b** to stabilize the Erlenmeyer shaped flask frame. Included in the Erlenmeyer shaped flask frame is a non-rigid polymeric gas permeable bag **109** connected to the rigid circular ring cap **106.** This embodiment further includes a stopper or a rigid screw type cap **122** for closure of the non-rigid gas permeable polymeric container or bag. The rigid screw type cap can further comprise a gas permeable section to provide removal of gases that rise to the top of the vessel such as carbon dioxide. For example, a silicone compound can be used for the gas permeable section to provide another section for diffusion of carbon dioxide from the interior of the bag.

The Erlenmeyer shaped flask frame **120** and circular cap **106** may be made from any rigid or substantially rigid polymeric materials described herein or otherwise suitable for such purposes, such as polyethylene (high-density or low-density polyethylene HDPE or LDPE), polyvinylchloride (PVC), polypropylene (PP), polystyrene (PS) including high impact polystyrene, polyamides (PA), acrylonitrile butadiene styrene (ABS), polycarbonate (PC), polycarbonate/acrylonitrile butadiene styrene (PC/ABS) etc.

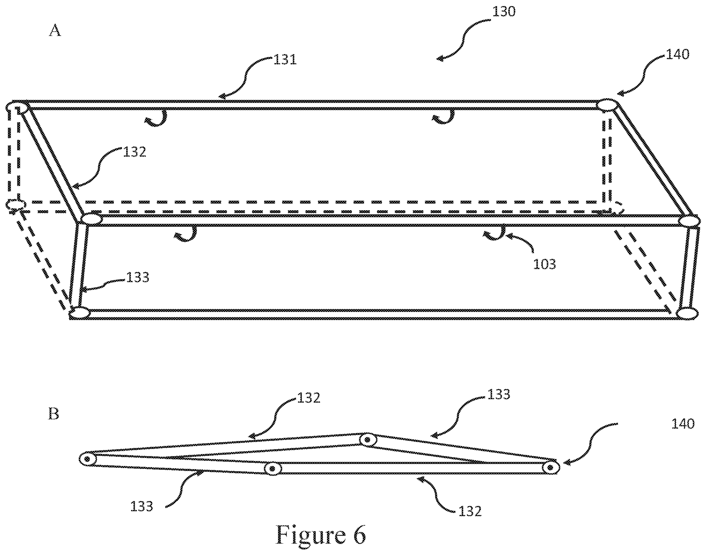

The present invention also provides for a foldable and reusable T-flask frame as shown in **Figure 6. Figure 6A** illustrates a three-dimensional rectangular frame **130** comprised of four two dimensional rectangular frames each fabricated of four rods that form two dimensional rectangular frames, wherein two of the rectangular frames comprise to rods of **131** lengths perpendicularly attached to two rods of **132** length connected on all corners to a hinging mechanism **140 and** two of the rectangular frames comprise to rods of **131** lengths perpendicularly attached to two rods of **133** length and connected on all corners to a hinging mechanism **140,** wherein all the frames have the same longitudinal length of **131** rod and rods **132** have an increased length relative to the **133** rods. **Figure 6** **B** shows the folding position of the foldable T-flask.

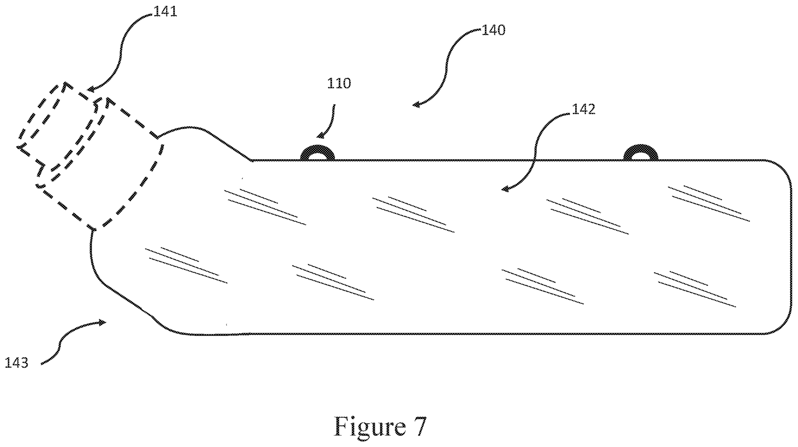

**Figure 7** illustrates a gas permeable polymeric pouch or bag **140** for insertion into the T-flask frame, the expandable position, shown in **Figure 6A****.** The pouch or bag **140** has a longitudinal length **142** that fits with the frame work of **130** and an angled extension **143** that include a closure cap **141**. The angled extension provides for easily access for filing the pouch. Preferably the angled extension **143** is fabricated from a sturdy polymeric material communicatively connected to the gas permeable section **142**. The pouch or bag can optionally include connecting rings **110** for connecting to **103** hooks shown in **Figure 6A**. Several gas-permeable materials have gas permeability sufficient to permit free passage of oxygen and carbon dioxide and can be selected from a group including, but not limited to, silicone compounds such as polydimethylsiloxane (PDMS), poly1-trimethylsilyl-1-propyne (PMSP), polypropylmethylsiloxane, polytrifluoropropylmethylsiloxane, and polyphenylmethylsiloxane; fluoroethylenepolypropylene, polyolefin, ethylene vinyl acetate copolymer, polyethylene, a cellulose acetate, a methacrylate, a hybrid material or a phthalate. Preferably, a silicone compound is used to fabricate the pouch.

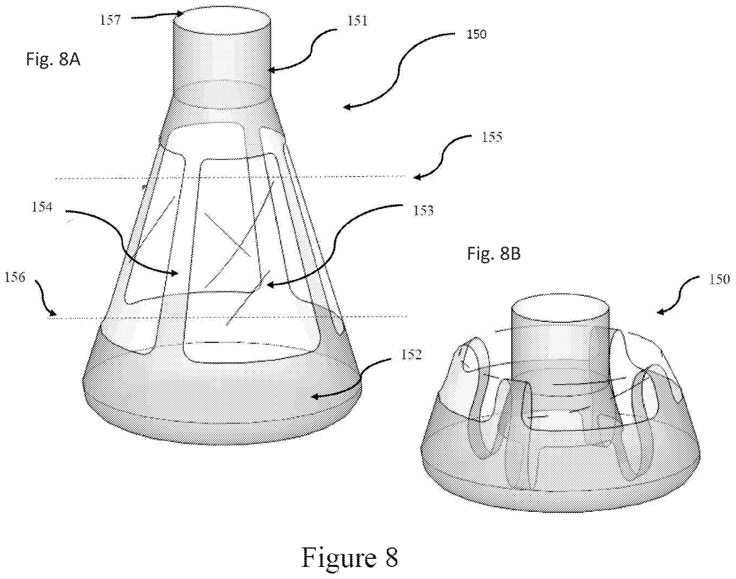

**Figure 8** **A** illustrates a collapsible Erlenmeyer flask shaped vessel **150** fabricated of both a gas permeable and a non-gas permeable polymeric film comprising: a flat base **152**, a generally conical body portion **153** and **154**, a generally tubular neck **151** having a diameter less than the body portion and a mouth of the tubular neck **157** for moving fluid into and out of the vessel. The generally conical body portion comprises strips of connecting gas permeable **153** and non-gas permeable polymeric **154** sections, wherein the non-gas permeable sections provide sufficient support for maintaining the conical shape and the gas permeable polymeric sections cover a greater area from that of the non-gas permeable polymeric sections. Both the gas permeable and non-gas permeable polymeric films are sufficiently strong to maintain shape of the vessel and sufficiently flexible to allow collapsibility of the vessel. Optionally the tubular neck and mouth of the vessel may be provided with a rigid sealable cap. The tubular neck can include a threaded section to provide the option of using a screw on cap to provide sealable closure.

The collapsibility of the vessel shown in **Figure 8** **B** is effected by collapsing the top section, shown above the **155** section line, into the middle section, shown above the **156** section line, and both in the bottom section of the vessel when the vessel is in an upright position. Thus, the sections of the vessel are collapsed vertically to form a collapsed configuration. In the compressed or collapsed position, the vessel is in a better arrangement for storage or transport. The vessel can easily be opened into an expanded vessel by simply moving the top, and middle section away from the bottom section.

The gas permeable polymeric film provides adequate rates of carbon dioxide and oxygen permeability while preventing passage of liquid. Several gas-permeable materials used in **153** section have gas permeability sufficient to permit free passage of oxygen and carbon dioxide and can be selected from a group including, but not limited to, silicone, fluoroethylenepolypropylene, polyolefin, ethylene vinyl acetate copolymer, polyethylene, a cellulose acetate, a methacrylate, a hybrid material or a phthalate. The non-gas permeable sections **154** can be any plastic commonly used in traditional culture vessels, or any other cell attachment material known to those skilled in the art.

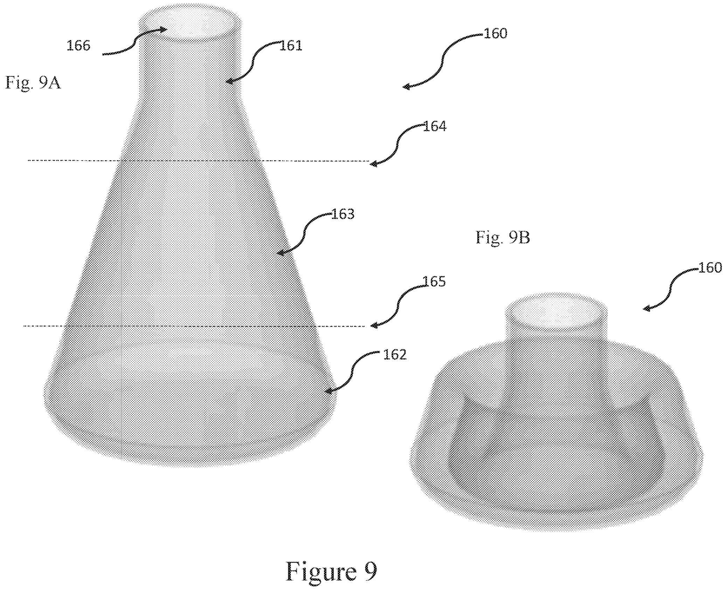

**Figure 9** **A** illustrates a collapsible Erlenmeyer flask shaped vessel **160** fabricated of a gas permeable e polymeric film comprising: a flat base **162**, a generally conical body portion **163**, a generally tubular neck **161** having a diameter less than the body portion and a mouth of the tubular neck **166** for moving fluid into and out of the vessel. The collapsible Erlenmeyer flask shaped vessel is fabricated of gas permeable film that is sufficiently strong to maintain shape of the vessel and sufficiently flexible to allow collapsibility of the vessel. Optionally the tubular neck and mouth of the vessel may be provided with a rigid sealable cap. The tubular neck can include a threaded section to provide the option of using a screw on cap to provide sealable closure.

The collapsibility of the vessel shown in **Figure 9** **B** is effected by collapsing the top section, shown above the **164** section line, into the middle section, shown above the **165** section line, and both in the bottom section of the vessel when the vessel is in an upright position. Thus, the sections of the vessel are collapsed vertically to form a collapsed configuration. In the compressed or collapsed position, the vessel is in a better arrangement for storage or transport. The vessel can easily be opened into an expanded vessel by simply moving the top, and middle section away from the bottom section.

The gas permeable polymeric film provides adequate rates of carbon dioxide and oxygen permeability while preventing passage of liquid. Several gas-permeable materials used in the vessel **160** have gas permeability sufficient to permit free passage of oxygen and carbon dioxide and can be selected from a group including, but not limited to, silicone, fluoroethylenepolypropylene, polyolefin, ethylene vinyl acetate copolymer, a cellulose acetate, a methacrylate or a phthalate.

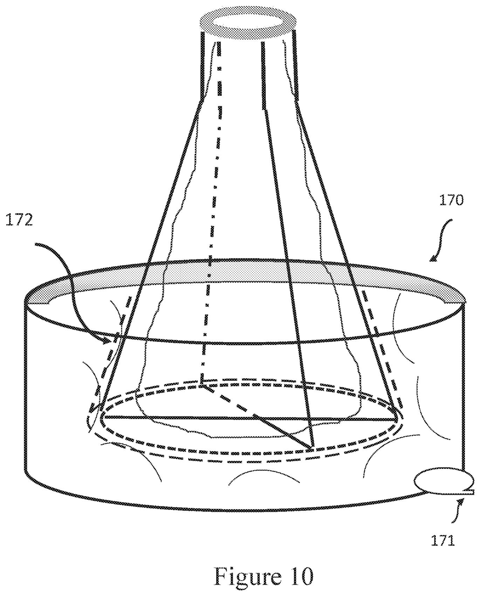

**Figure 10** shows an inflatable support holder **170** for a flask of the present invention. Upon inflation, the holder surrounds the flask and provides support by inflating a rim **172** that surrounds the flask. The holder can be fabricated of polymeric material including but not limited to include polystyrene, polymethylmethacrylate, polyvinyl chloride, polycarbonate, polysulfone, polystyrene copolymers, fluoropolymers, polyesters, polyamides, polystyrene butadiene copolymers, fully hydrogenated styrenic polymers, polycarbonate PDMS copolymers, and polyolefins such as polyethylene, polypropylene, polymethyl pentene, polypropylene copolymers and cyclic olefin copolymers, and the like.

The inflatable holder defines a collapsible, substantially fluid-tight container. The container is formed to have a reduced neck portion formed by the inflated rim **172**, a flat bottom for stability and having a valve **171** therein for filling the support with air. The reduced neck fits around the flask body. The valve may comprise a stem extending outward from a side of the support and a removable plug for sealing the stem. Ordinarily, about 4 to 10 pounds per square inch of air is sufficient to properly inflate the structure.

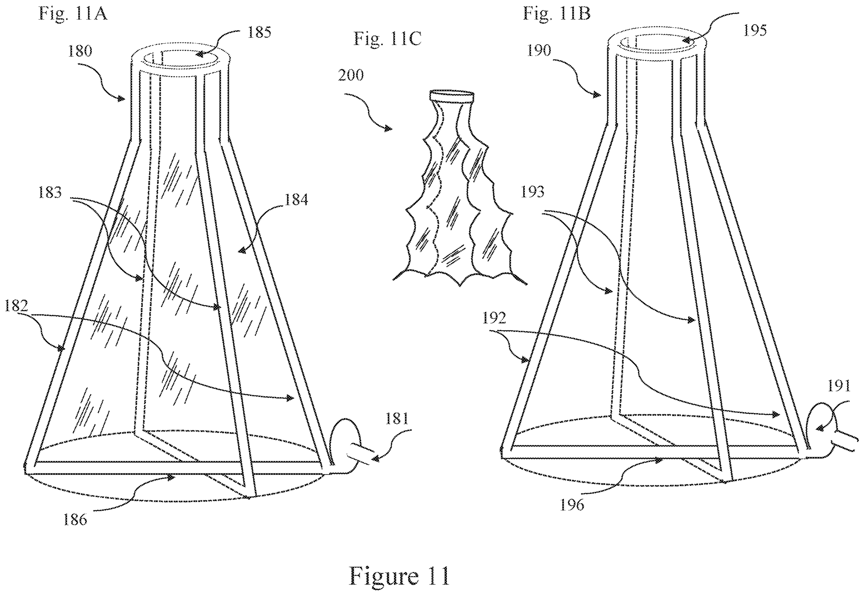

**Figure 11** **A** illustrates an inflatable flask structure **180** wherein the structures comprise a plurality of pneumatically interconnected, elongate inflatable tubes **182** and **183** positioned in spaced-apart relation to provide a conical shaped structure for being inflated in unison, said tubes defining a flask type structure have an opening ring structure **185** at the proximal end and flat bottom structure **186** at the distal end. Further included is a valve means **181** for inflating the tubes. Still further this embodiment comprises a plurality of wall panels **184** attached from and between adjacent tubes to define an enclosure of the flask, wherein the plurality of wall panels **184** are fabricated from a gas permeable polymeric material and wherein the inflatable tubes are fabricated from an impermeable and flexible polymeric material.

The inflatable tubes are preferably fabricated of a flexible gas impermeable material such as a rubberized material, polymeric material or a thermoplastic sheet material, wherein the material has sufficient density to resist passage of air under pressure. When inflated the flask sharped structure is formed. Valve means **181** is provided for inflating the tubes, wherein the valve means includes a manifold into which all of the tubes interconnect and the tubes are connected with an air pump for inflation. Ordinarily, about 4 to 10 pounds per square inch of air is sufficient to properly inflate the structure and maintain the stability of the structure.

When air pressure is reduced the structure **200** collapses as shown in **Figure 11** **C**.

**Figure 11** **B** illustrates an inflatable flask structure **190** wherein the structures comprise a plurality of pneumatically interconnected, elongate inflatable tubes **192** and **193** positioned in spaced-apart relation to provide a conical shaped structure for being inflated in unison, said tubes defining a flask type structure have an opening ring structure **195** at the proximal end and flat bottom structure **196** at the distal end. Further included is a valve means **191** for inflating the tubes. The inflatable tubes are fabricated from a gas impermeable and flexible polymeric material.

The inflatable tubes are preferably fabricated of a flexible gas impermeable material such as a rubberized material, polymeric material or a thermoplastic sheet material, wherein the material has sufficient density to resist passage of air under pressure. When inflated the flask sharped structure is formed. Valve means **191** is provided for inflating the tubes, wherein the valve means includes a manifold into which all of the tubes interconnect and the tubes are connected with an air pump for inflation. Ordinarily, about 4 to 10 pounds per square inch of air is sufficient to properly inflate the structure and maintain the stability of the structure.

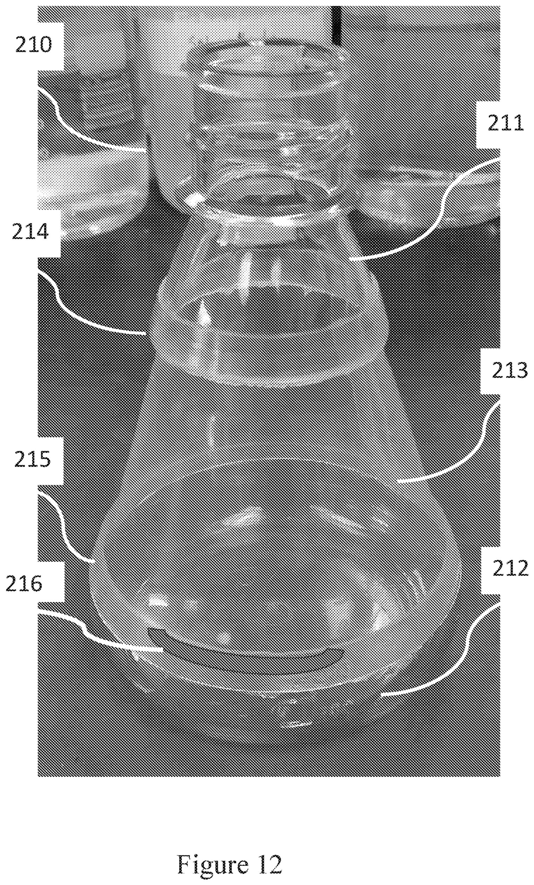

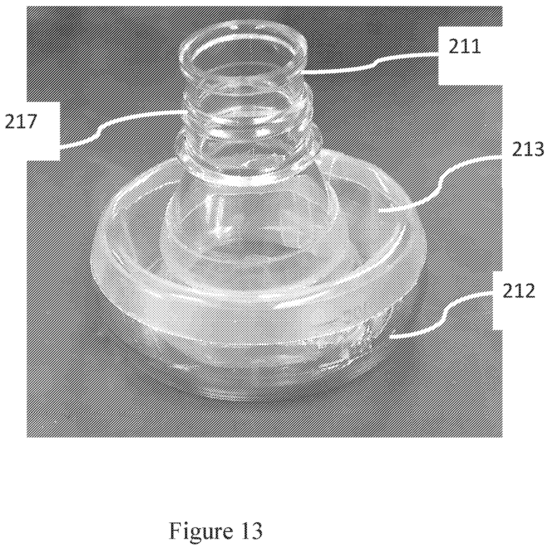

**Figure 12** illustrates another embodiment, providing a collapsible conical shaped Erlenmeyer flask **210** comprising three sections, wherein the first section is a non-flexible neck section **211**, the second section comprises a non-flexible flat bottom bowl section **212** and a third section comprising a flexible sleeve **213** that is positioned between the first and second section and connected to each to form a sealed collapsible Erlenmeyer flask, wherein the flexible sleeve **213** collapsibly folds upon itself to position the non-flexible neck section **211** closer to the non-flexible flat bottom bowl section **212**, as shown in **Figure 13**. In the folding process, the neck section and substantially all of the flexible sleeve are contained with the non-flexible flat bottom bowl section, thereby reducing the size for easy storage.

The flexible sleeve comprises a first opening having smaller cross-section diameter than a second opening, wherein the first and second opening are positioned at opposite ends of the flexible sleeve and where in the first opening is connect to the non-flexible neck section at **214** and the second opening is connect to the non-flexible flat bottom bowl section **215**. The flexible sleeve is connected to both the non-flexible neck section and the non-flexible flat bottom bowl section by meeting the edges of each and overlapping a bottom section of the non-flexible neck section **214** and the top section of the non-flexible flat bottom bowl section **215**. The flexible sleeve has the ability and elasticity to stretch over the bottom edge of the neck section and the top edge of the non-flexible flat bottom bowl section. Additionally, a sealing band can be connected to flexible sleeve at the junction point of the overlapping sleeve section to the neck section and flat bottom bowl section. The sealing bands may be a narrow metal strip **216**, wherein the bands can be slipped over the neck section for positioning at the appropriate junction points. In **Figure 12**, the sealing band is shown in a truncated form just to provide preferred positioning.

The non-flexible material used to fabricate the neck section and flat-bottom bowl section can be selected from glass or a polymer, and preferably transparent. A hard nonflexible polymer includes but is not limited to High-density polyethylene(HDPE), Polypropylene (PP), Polycarbonate, Polyvinyl chloride (PVC), Polystyrene (PS), Nylon, nylon 6, nylon 6,6, Teflon (Polytetrafluoroethylene) and Thermoplastic polyurethanes (TPU). Preferably the non-flexible and hard material is heat resistant.

The flexible polymeric material acceptable to fabricate the flexible sleeve may include any flexible gas permeable film, such as discussed above and preferably a silicone material.

The neck section **211** is preferably configured to include a top and bottom section **214** where the top section is threaded **217** to provide the ability to connect a screw type cap to enclose the collapsible conical shaped Erlenmeyer flask. The neck section can include a threaded section to provide the option of using a screw on cap to provide sealable closure.

The flexible sleeve **213** is conical shaped wherein the first opening is of sufficient diameter to overlap the bottom edge of the neck section **214** and the second opening opposite the first opening has a larger diameter than the first opening is of sufficient diameter to overlap the top edge of the flat-bottom bowel section. **215**. Notably the first and second opening are position on a plane that is perpendicular to the longitudinal axis of the flexible sleeve.

### Testing of Gas Permeability of Silicon Containing Collapsible Flasks

Shake flasks are ubiquitous in fermentation, however, there is typically a paucity of oxygen along with carbon dioxide buildup during cell growth in a shake flask. This is due to insufficient gas transfer between the environment and flask during aerobic growth. The present invention provides plastic shake flasks with gas permeable silicone membrane walls with portable, non-collapsible and collapsible designs. As shown in **Figures 12** **and** **13****,** the silicone walls allow for improved transfer of oxygen and carbon dioxide to and from the environment increasing availability of oxygen for cell growth while minimizing carbon dioxide buildup. *E.coli* fermentations were conducted in both commercial standard shake flasks and modified gas permeable flasks. The important factors that affect cell growth, namely dissolved oxygen and dissolved carbon dioxide were measured using noninvasive sensors, and cell growth was compared.

### Materials and methods

For these experiments in the flask as shown in Figures 12 and 13, a noninvasive method of measuring dissolved oxygen (DO) was used wherein such a method included using an optical sensor. The oxygen sensitive component was a thin film attached to the bottom of the flask which responds differently to light of certain wavelengths at different dissolved oxygen concentrations. The device had been tested to be accurate up to 60% dissolved oxygen concentration in previous studies conducted by the inventors. For dissolved carbon dioxide (DCO₂), a silicone sampling loop was inserted into the flask and then flushed with ambient air until the prior carbon dioxide in the loop was completely expelled. This was done after each measurement. The gas dissolved in the solution was then allowed to recirculate through the gas permeable silicone membrane into the sensor. The silicone tubing had a small spring within that prevented sharp folds and kinks and was also enclosed by a bigger spring to ensure that it hugged the walls of the shake flask and stayed in place at the bottom during trials. All trials were conducted for 24 hours at a temperature of 30° C at 250 rpm in an incubator with a Lysogeny broth (LB) medium at a total volume of 150 ml. 40 standardized batches of 300µl *E.Coli* samples were first prepared and then frozen in 1ml plastic containers of which 250µl was used for each trial. They were thawed each time a trial was run; this was done to eliminate variability between *E.Coli* samples by ensuring that each sample had been frozen and thawed exactly one time before being used in the trial. 250µl of Ampicillin- a beta lactam antibiotic- was also used to ensure that all the *E.Coli* in the sample was uniform and of the same kind. Silicone films of two thicknesses were studied- 0.3 and 0.1mm. Silicone epoxy was used to bind the silicone to the plastic shake flask and left to cure for 24 hours. Distilled water (DI) was then introduced into the flask to check for leakages, which if present, were plugged with more silicone epoxy. Testing on the 0.1mm thickness silicone was conducted without autoclaving to ensure stability of the film.

### Results

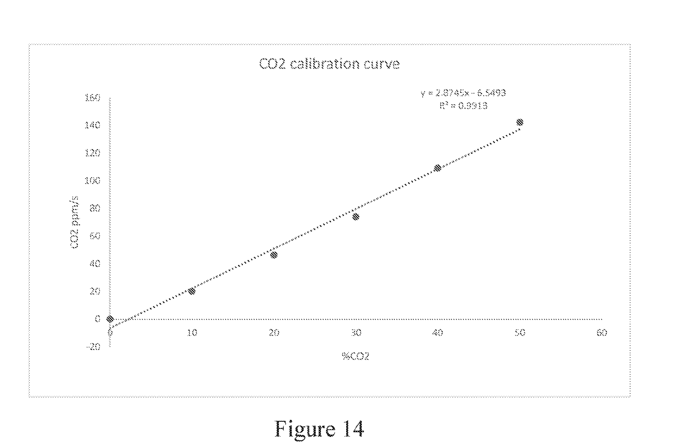

Carbon dioxide was calibrated using known concentrations of CO₂ gas. **Figure 14** is an example of a CO₂ calibration curve where the y-axis is the slope of the CO₂ sampling curve (ppm vs time) and the x-axis is the known gas percentage of CO₂ passed through a reference LB broth solution.

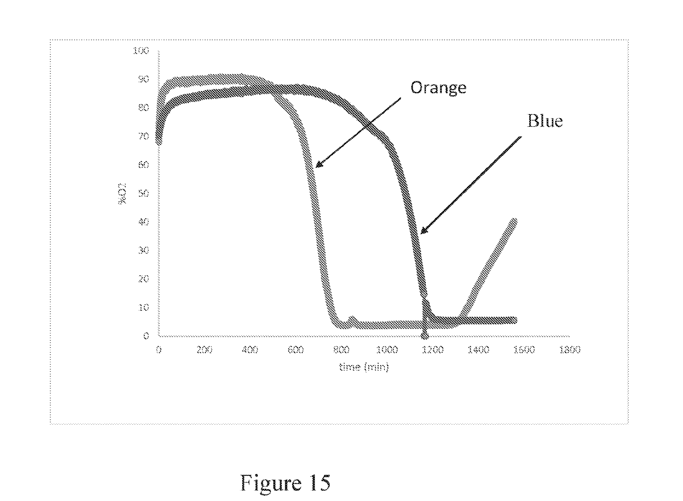

It was noticed that using compressible silicone walls of 0.3mm sped up the rate of oxygen consumption and by extension, *E .Coli* growth and demonstrated an increased availability of oxygen after the growth phase as compared to the polymer flask, as shown in **Figure 15**. Of the two silicone film thicknesses studied- 0.3 and 0.1mm- it was hypothesized that the 0.1mm film would provide greater gas permeability than the 0.3mm.

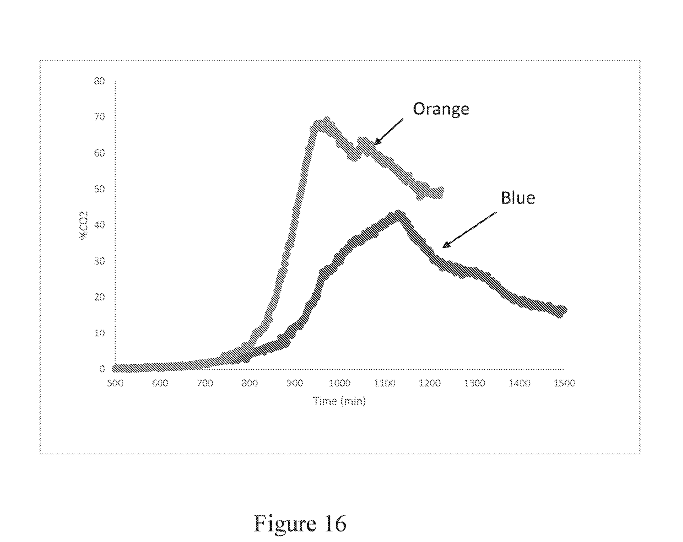

Dissolved carbon dioxide (DCO₂) also followed the expected trend where the no silicone plastic shake flask had a higher concentration of DCO₂ than the silicone walled variant as shown in **Figure 16****.**

### Conclusion

The results show that the oxygen limitation usually occurring in standard shake flasks was significantly improved in the modified shake flasks with a silicone section and cell growth was similarly enhanced. There was an increase in available DO and a reduction in DCO₂ with 0.3mm gas permeable silicone walls as opposed to plane plastic walls. As 0.1mm silicone is thinner and more permeable than the 0.3mm variant, it can be hypothesized that there would be greater available DO and lesser DCO₂.

### Further aspects and embodiments of the invention

Further aspects and embodiments of the invention are defined in the following clauses:
Clause 1. A conical shaped flask comprising:
   a conical shaped frame comprised of two rigid rod-type supports each having proximal end forming a flat base and two opposing upwardly extending distal ends to form a generally conical body, wherein the flat base of the two rigid supports are connected at a central pivot point thereby allowing the two rigid rod-type supports to form a conical body portion when separated furthest from each other and foldable into an essentially flat frame when adjacent;
   a rigid circular ring cap forming a tubular neck for placement and connecting thereto the upwardly extending distal ends to stabilize the conical shaped frame, wherein the rigid circular ring cap has an exterior and interior edge; and
   a non-rigid gas permeable polymeric container or bag fabricated of a gas permeable polymeric material and sized to fit within the conical body portion and attached to the interior edge of the circular ring cap for positioning therein.
Clause 2. The conical shaped flask according to clause 1, wherein the rigid circular ring cap comprises four recesses/slots equally distributed within the bottom of the circular ring cap thereby providing connection to the upwardly extending distal ends.
Clause 3. The conical shaped flask according to clause 1, wherein the two rigid rod-type supports comprise hook type attachments for securing the non-rigid gas permeable polymeric container or bag to the conical body portion.
Clause 4. The conical shaped flask according to clause 1, further comprising a top stopper or a rigid screw type cap for closure of the non-rigid gas permeable polymeric container or bag.
Clause 5. The conical shaped flask according to clause 1, wherein the circular ring cap is a cylindrical ring thereby forming a neck wherein the neck has a uniform internal diameter for its entire height.
Clause 6. The conical shaped flask according to clause 1, wherein the gas permeable polymeric material is selected from the group consisting of silicone, fluoroethylenepolypropylene, polyolefin, ethylene vinyl acetate copolymer, polyethylene, a cellulose acetate, a methacrylate, a hybrid material and a phthalate.
Clause 7. The conical shaped flask according to clause 1, wherein the two rigid rod-type supports are fabricated of a rigid material or substantially rigid material including a polymer or metal.
Clause 8. A T-flask of comprising:
   four rectangular frames each comprising four rigid support members pivotably attached to each other by corner hinges to form a two dimensional rectangular shape, wherein the four rigid support members are fabricated of a rigid material or substantially rigid material, wherein the four rectangular frames are connected through the corner hinges and pivot with respect to each other to an expanded configuration to form a three dimensional rectangular frame and a collapsed configuration wherein the four rectangular frames lie approximately parallel to each other; and
   a non-rigid gas permeable polymeric container or bag comprising a chamber, a neck connected to the chamber for introducing fluids into the chamber, a closure cap attachable to the neck, wherein the chamber is sized to fit within the three dimensional rectangular structure, wherein the chamber of the non-rigid gas permeable polymeric container or bag is communicatively connected to the four rectangular frames, and wherein the neck and closure cap are not positioned within the rectangular frame.
Clause 9. The T-flask according to clause 8, wherein the hinges include a locking mechanism to support the three dimensional rectangular frame in expanded configuration.
Clause 10. The T-flask according to clause 8, wherein the four rigid support members comprise hook type attachments for securing the non-rigid gas permeable polymeric container or bag to the three- dimensional rectangular frame.
Clause 11. The T-flask according to clause 8, wherein the non-rigid gas permeable polymeric container or bag is fabricated of a polymeric material selected from the group consisting of silicone, fluoroethylenepolypropylene, polyolefin, ethylene vinyl acetate copolymer, a cellulose acetate, a methacrylate and a phthalate.
Clause 12. The conical shaped flask according to clause 8, wherein the wherein the rigid material or substantially rigid material is a rigid polymer or metal.
Clause 13. A collapsible Erlenmeyer flask shaped vessel fabricated of a gas permeable polymeric film comprising: a flat base, a generally conical body portion, a generally tubular neck having a diameter less than the body portion, wherein the tubular neck comprises an opening for moving fluid into and out of the vessel, wherein gas permeable polymeric film is sufficiently strong to maintain shape of the vessel and sufficiently flexible to allow collapsibility of the vessel.
Clause 14. The collapsible Erlenmeyer flask shaped vessel according to clause 13, wherein a rigid sealable cap is optionally connected to the tubular neck of the vessel.
Clause 15. The collapsible Erlenmeyer flask shaped vessel according to clause 13, wherein the vessel comprises a top section, middle section and bottom section, and wherein the collapsibility of the vessel is effected by collapsing the top section into the middle section and both of same into the bottom section of the vessel when the vessel is in an upright position.
Clause 16. The collapsible Erlenmeyer flask shaped vessel according to clause 15, where the sections of the vessel are collapsed vertically to form a compressed configuration.
Clause 17. The collapsible Erlenmeyer flask shaped vessel according to clause 13, wherein the gas permeable polymeric film provides adequate rates of carbon dioxide and oxygen permeability while preventing passage of liquid.
Clause 18. The collapsible Erlenmeyer flask shaped vessel according to clause 13, where the wherein the gas permeable polymeric film is selected from the group consisting of silicone, fluoroethylenepolypropylene, polyolefin, polyethylene, ethylene vinyl acetate copolymer, a cellulose acetate, a methacrylate, a hybrid material and a phthalate.
Clause 19. A collapsible Erlenmeyer flask shaped vessel fabricated of both a gas permeable and a non-gas permeable polymeric film comprising:
   a flat base, a generally conical body portion, a generally tubular neck having a diameter less than the body portion and a mouth of the tubular neck for moving fluid into and out of the vessel, wherein the generally conical body portion comprises strips of connecting gas permeable and non-gas permeable polymeric sections, wherein the non-gas permeable sections provide sufficient support for maintaining the conical shape and the gas permeable polymeric sections cover a greater area from that of the non-gas permeable polymeric sections.
Clause 20. The collapsible Erlenmeyer flask shaped vessel according to clause 19, wherein the gas permeable and non-gas permeable polymeric films are sufficiently strong to maintain the shape of the vessel and sufficiently flexible to allow collapsibility of the vessel.
Clause 21. The collapsible Erlenmeyer flask shaped vessel according to clause 19, wherein a rigid sealable cap is optionally connected to the tubular neck of the vessel.
Clause 22. The collapsible Erlenmeyer flask shaped vessel according to clause 19, wherein the vessel comprises a top section, middle section and bottom section, and wherein the collapsibility of the vessel is effected by collapsing the top section into the middle section and both of same into the bottom section of the vessel when the vessel is in an upright position.
Clause 23. The collapsible Erlenmeyer flask shaped vessel according to clause 22, where the sections of the vessel are collapsed vertically to form a compressed configuration.
Clause 24. The collapsible Erlenmeyer flask shaped vessel according to clause 19, wherein the gas permeable polymeric film provides adequate rates of carbon dioxide and oxygen permeability while preventing passage of liquid.
Clause 25. The collapsible Erlenmeyer flask shaped vessel according to clause 19, wherein the gas permeable polymeric film is selected from the group consisting of silicone, fluoroethylenepolypropylene, polyolefin, polyethylene, ethylene vinyl acetate copolymer, a cellulose acetate, a methacrylate, a hybrid material and a phthalate.
Clause 26. The collapsible Erlenmeyer flask shaped vessel according to clause 19, wherein the non-gas permeable sections can be any plastic commonly used in traditional culture vessels, or any other cell attachment material known to those skilled in the art.
Clause 27. An inflatable holder for supporting a flask comprising:
   a flexible bag body having an open end wherein the open end comprises an adjustable valve for introducing compressed air into the flexible bag, wherein the adjustable valve is sealable to retain the compressed air after inflating, wherein the bag is sized sufficiently for providing an inflatable extendible rim to encompass and provide support for an inserted flask.
Clause 28. The inflatable holder for supporting a flask according to clause 27, wherein the flexible bag body is fabricated of a flexible gas impermeable material.
Clause 29. An inflatable flask structure comprising:
   a plurality of pneumatically interconnected, elongate inflatable tubes positioned in spaced-apart relation to provide a conical shaped structure for being inflated in unison, said tubes defining a flask type structure have an opening at the proximal end and flat bottom structure at the distal end;
   valve means for inflating the tubes; and
   optionally a plurality of wall panels attached from and between adjacent tubes to define an enclosure of the flask, wherein the plurality of wall panels are fabricated from a gas permeable polymeric material and wherein the inflatable tubes are fabricated from an impermeable flexible polymeric material.
Clause 30. A collapsible conical shaped Erlenmeyer flask comprising three sections, wherein the first section is a non-flexible neck section, the second section comprises a non-flexible flat bottom bowl section and a third section comprising a flexible sleeve that is positioned between the first and second section and connected to each to form a sealed collapsible Erlenmeyer flask, wherein the flexible sleeve collapsible folds upon itself to position the non-flexible neck section closer to the non-flexible flat bottom bowl section.
Clause 31. The collapsible conical shaped Erlenmeyer flask according to clause 30, wherein, the neck section and substantially all of the flexible sleeve are folded and contained with the non-flexible flat bottom bowl section, thereby reducing the size for easy storage.
Clause 32. The collapsible conical shaped Erlenmeyer flask according to clause 30, wherein the flexible sleeve comprises a first opening having smaller cross-section diameter than a second opening, wherein the first and second opening are positioned at opposite ends of the flexible sleeve and where in the first opening is connect to the non-flexible neck section and the second opening is connect to the non-flexible flat bottom bowl section.
Clause 33. The collapsible conical shaped Erlenmeyer flask according to clause 30, wherein the flexible sleeve is connected to both the non-flexible neck section and the non-flexible flat bottom bowl section by meeting the edges of each and overlapping a bottom section of the non-flexible neck section and the top section of the non-flexible flat bottom bowl section.
Clause 34. The collapsible conical shaped Erlenmeyer flask according to clause 30, further comprising a sealing band connected to flexible sleeve at a junction point of the overlapping sleeve section to the neck section and flat bottom bowl section.
Clause 35. The collapsible conical shaped Erlenmeyer flask according to clause 30, wherein the non-flexible material used to fabricate the neck section and flat-bottom bowl section can be selected from glass or a polymer.
Clause 36. The collapsible conical shaped Erlenmeyer flask according to clause 30, wherein the neck section is configured to include a threaded section o provide the ability to connect a screw type cap to enclose the collapsible conical shaped Erlenmeyer flask.
Clause 37. The collapsible conical shaped Erlenmeyer flask according to clause 30, wherein the flexible sleeve is fabricated of a gas permeable silicone material.

## Claims

1. A collapsible Erlenmeyer flask shaped vessel comprising:
a flat base;
a generally conical body portion; and
a generally tubular neck having a diameter less than the body portion and a mouth of
the tubular neck for moving fluid into and out of the vessel,
wherein the vessel is:
(I) fabricated of both a gas permeable and a non-gas permeable polymeric film and the generally conical body portion comprises strips of connecting gas permeable and non-gas permeable polymeric sections, wherein the non-gas permeable sections provide sufficient support for maintaining the conical shape and the gas permeable polymeric sections cover a greater area from that of the non-gas permeable polymeric sections; or
(II) fabricated of a gas permeable polymeric film, wherein gas permeable polymeric film is sufficiently strong to maintain shape of the vessel and sufficiently flexible to allow collapsibility of the vessel.

2. The collapsible Erlenmeyer flask shaped vessel according to claim 1, option (I), wherein the gas permeable and non-gas permeable polymeric films are sufficiently strong to maintain the shape of the vessel and sufficiently flexible to allow collapsibility of the vessel.

3. The collapsible Erlenmeyer flask shaped vessel according to claim 1, wherein a rigid sealable cap is optionally connected to the tubular neck of the vessel.

4. The collapsible Erlenmeyer flask shaped vessel according to claim 1, wherein the vessel comprises a top section, middle section and bottom section, and wherein the collapsibility of the vessel is effected by collapsing the top section into the middle section and both of same into the bottom section of the vessel when the vessel is in an upright position.

5. The collapsible Erlenmeyer flask shaped vessel according to claim 4, wherein the sections of the vessel are collapsed vertically to form a compressed configuration.

6. The collapsible Erlenmeyer flask shaped vessel according to claim 1, wherein the gas permeable polymeric film provides adequate rates of carbon dioxide and oxygen permeability while preventing passage of liquid.

7. The collapsible Erlenmeyer flask shaped vessel according to claim 1, wherein the gas permeable polymeric film is selected from the group consisting of silicone, fluoroethylenepolypropylene, polyolefin, polyethylene, ethylene vinyl acetate copolymer, a cellulose acetate, a methacrylate, a hybrid material and a phthalate.

8. The collapsible Erlenmeyer flask shaped vessel according to claim 1, option (I), wherein the non-gas permeable polymeric film is selected from the group consisting of polystyrene, polymethylmethacrylate, polyvinyl chloride, polycarbonate, polysulfone, polystyrene copolymers, fluoropolymers, polyesters, polyamides, polystyrene butadiene copolymers, fully hydrogenated styrenic polymers, polycarbonate PDMS copolymers, and polyolefins such as polyethylene, polypropylene, polymethyl pentene, polypropylene copolymers and cyclic olefin copolymers.
